(19) Europäisches Patentamt
European Patent Office
Office européen des brevets

(11) Publication number: 0 327 251 A1

# EUROPEAN PATENT APPLICATION

(21) Application number: 89300726.0

(51) Int. Cl.4: C07D 401/12 , A01N 47/36

(22) Date of filing: 26.01.89

(30) Priority: 01.02.88 US 151304

(43) Date of publication of application: 09.08.89 Bulletin 89/32

(84) Designated Contracting States: ES GR

(71) Applicant: E.I. DU PONT DE NEMOURS AND COMPANY
Legal Department 1007 Market Street
Wilmington Delaware 19898(US)

(72) Inventor: Liang, Paul Hsaio-Tseng
3428 Pebble Beach Drive
Wilmington Delaware, 19898(US)

(74) Representative: Hildyard, Edward Martin et al
Frank B. Dehn & Co. European Patent Attorneys Imperial House 15-19 Kingsway
London WC2B 6UZ(GB)

(54) **Herbicides for control of blackgrass.**

(57) Compounds of the formula

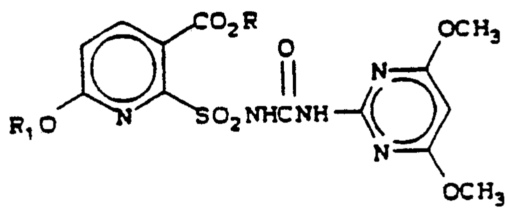

I

wherein
R is $C_1$-$C_3$ alkyl; and
$R_1$ is $C_1$-$C_2$ fluoroalkyl;
and their agriculturally suitable salts exhibit herbicidal activity and may be used inter alia for controlling blackgrass in cereal crops.

EP 0 327 251 A1

## HERBICIDES FOR CONTROL OF BLACKGRASS

### FIELD OF THE INVENTION

Blackgrass is controlled by applying certain compounds described herein, especially in cereal crops.

### DESCRIPTION OF RELATED ART

U.S. 4,435,206 discloses herbicidal sulfonamides of the formula

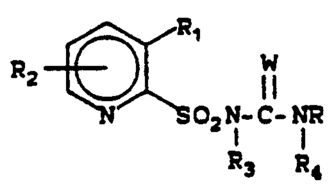

wherein
R is

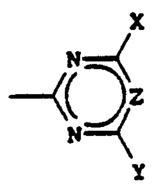 , 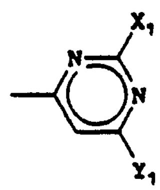 or 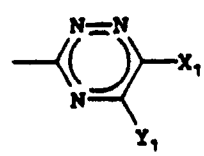 ;

$R_1$ is H, Cl, Br, F, $C_1$-$C_4$ alkyl, $C_1$-$C_4$ alkoxy, $C_1$-$C_4$ alkylthio, $NO_2$, $CF_3$, $COOR_5$ or $SO_2NR_6R_7$;
$R_2$ is H, Cl, Br or $CH_3$;
$R_3$ and $R_4$ are independently H or $CH_3$; and $R_5$ is $C_1$-$C_6$ alkyl, $C_3$-$C_6$ alkenyl, $CH_2CH_2OCH_3$, $CH_2CH_2OCH_2CH_3$, $CH_2CH_2CH_2OCH_3$ or $CH_2CH_2Cl$.

Although this reference discloses certain pyridinesulfonylureas, it does not describe the pyridinesulfonylureas of the instant invention or their use for controlling blackgrass.

### SUMMARY OF THE INVENTION

This invention pertains to the novel method-of-use of compounds of Formula I as preemergence and/or postemergence herbicides for the control of blackgrass and to agriculturally suitable compositions containing these compounds for the control of blackgrass.

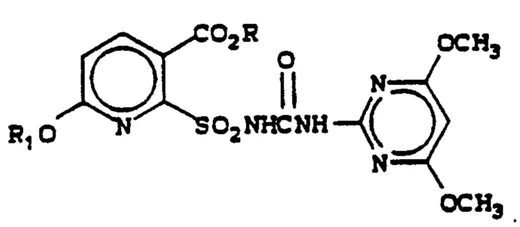 I

wherein
R is $C_1$-$C_3$ alkyl; and

$R_1$ is $C_1$-$C_2$ fluoroalkyl;

and their agriculturally suitable salts.

In the above definition the term "$C_1$-$C_3$ alkyl" includes $CH_3$, $CH_2CH_3$, $CH_2CH_2CH_3$ and $CH(CH_3)_2$. The term "$C_1$-$C_2$ fluoroalkyl" includes methyl and ethyl which are partially or fully substituted with fluorine atoms.

This invention also comprises compounds of Formula I wherein R and $R_1$ are as defined above. These include compounds where when R is $CH_3$ or $CH_2CH_3$, then $R_1$ is other than $CH_2CF_3$, $CF_2H$ and $CF_3$.

The preferred embodiments of the present invention are the use of the compounds of Formula I where $R_1$ is $CF_2H$ or $CH_2CF_3$, especially $CH_2CF_3$, for controlling blackgrass and agriculturally suitable compositions containing these compounds for the control of blackgrass in cereal crops.

Particularly preferred for reason of greatest herbicidal efficacy combined with cereal crop tolerance are the use of methyl 2-[[[[(4,6-dimethoxypyrimidin-2-yl)amino]carbonyl]amino]sulfonyl]-6-(2,2,2-trifluoroethoxy)-3-pyridinecarboxylate (Formula I: R is $CH_3$, and $R_1$ is $CH_2CF_3$; melting Point 158-162 °C) for controlling blackgrass and agriculturally suitable compositions containing this compound for the control of blackgrass.

## DETAILED DESCRIPTION OF THE INVENTION

### Synthesis

Compounds of Formula I can be synthesized by one or more of the general methods described in U.S. 4,456,469, or by the reactions shown in Equations 1a and 1b. The reaction of sulfonamides II with the phenyl ester of the appropriate carbamic acid IIIa in the presence of various bases such as a tertiary amine base, 1,8-diazabicyclo[5.4.0]-undec-7-ene (DBU) for example, is shown in Equation 1a.

Equation 1a

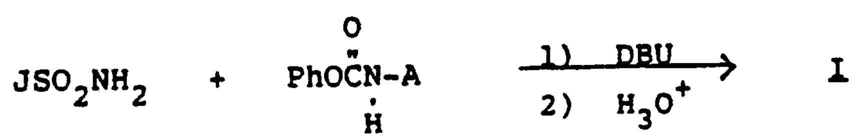


II IIIa

wherein

J is 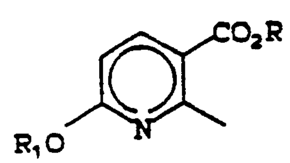 ,

A is 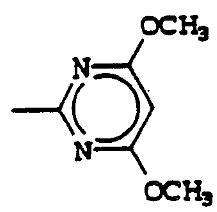 ; and

R and $R_1$ are as previously defined.

The reaction shown in Equation 1a is best carried out at 25 °C in a solvent such as dioxane or

acetonitrile for 1-2 hours under an inert atmosphere as described in European Patent Application No. 70,804 (published January 26, 1983). The desired products of Formula I can be conveniently isolated by acidifying the reaction solution with aqueous hydrochloric acid. Alternatively, the aqueous layer can be extracted with a solvent such as methylene chloride or ethyl acetate. Drying and evaporation of the solvent affords the desired products. The phenyl carbamate can be synthesized by treatment of the corresponding heterocyclic amine of Formula $A\text{-}NH_2$ with diphenyl carbonate or phenyl chloroformate in the presence of a base such as sodium hydride, pyridine, or potassium carbonate with a catalytic amount of 4-dimethylaminopyridine. The mixture is stirred at temperatures between 25° and 65° C in a suitable solvent such as tetrahydrofuran for 12-36 hours.

Compounds of Formula I can also be synthesized by the reaction of the appropriate arylsulfonylcarbamates of Formula IIIc with the heterocyclic amine IIId as shown in Equation 1b. This reaction can be carried out in the presence of various bases as described in Equation 1a, or by simply refluxing in an appropriately high boiling solvent such as dioxane. The arylsulfonylcarbamates can be synthesized by the reaction of sulfonamide II with phenyl or alkyl carbonates IIIb (or the corresponding chloroformates) in the presence of a variety of bases.

Equation 1b

$$\underset{\text{II}}{JSO_2NH_2} + \underset{\text{IIIb}}{R_2O\overset{\overset{O}{\|}}{C}OR_2} \longrightarrow \underset{\text{IIIc}}{JSO_2NHCOR_2}$$

$$\text{IIIc} + \underset{\text{IIId}}{A\text{-}NH_2} \longrightarrow \text{I}$$

wherein

$R_2$ is phenyl or $C_1$-$C_4$ alkyl; and

J and A are as previously defined.

The required sulfonamides of Formula II can be synthesized by either one of the methods shown below in Equations 2 and 3.

Equation 2 depicts the reaction of sulfonyl chlorides of Formula IV with ammonia to give sulfonamides of Formula II.

Equation 2

$$\underset{\text{IV}}{JSO_2Cl} \xrightarrow{NH_3} \underset{\text{II}}{JSO_2NH_2}$$

wherein

J is as Previously defined.

The amination of Equation 2 is conveniently effected by adding at least two molar equivalents of either anhydrous ammonia or concentrated ammonium hydroxide to a solution of the sulfonyl chloride IV in a suitable solvent such as diethyl ether, tetrahydrofuran or methylene chloride at temperatures between -30° and 25° C. The desired sulfonamides of Formula II are isolated either by filtration, in which case the by-product ammonium chloride is removed by washing with water, or extraction into a suitable organic solvent such as methylene chloride or ethyl acetate. Drying and evaporation of the solvent then affords the products II which are usually sufficiently pure to be carried directly on to the next step.

Sulfonamides of Formula II can also be prepared as shown in Equation 3 by treatment of the

corresponding N-t-butylsulfonamides VI with an appropriate acid such as trifluoroacetic (TFA), polyphosphoric (PPA), or p-toluenesulfonic acid (p-TSA).

Equation 3

$$JSO_2NH\text{-}\underline{t}\text{-}Bu \xrightarrow[\underline{p}\text{-TSA}]{\text{TFA, PPA or}} JSO_2NH_2$$

VI → II

wherein

J is as previously defined.

The reaction of Equation 3 is conveniently carried out by stirring a solution of the compound of Formula VI in excess trifluoroacetic acid (approximately 0.3M) at about 25°C for 1-72 hours. The desired sulfonamides of Formula II are then isolated by removal of the volatiles in vacuo and crystallization from a suitable solvent such as diethyl ether, 1-chlorobutane, or ethyl acetate. Alternatively, the N-t-butylsulfonamides of Formula VI can be treated with a catalytic amount of p-toluenesulfonic acid monohydrate in a solvent such as toluene or xylenes at reflux temperature for 1-6 hours. The desired products are then isolated in a manner analogous to the one described above. For use of polyphosphoric acid in the deprotection of N-t-butylsulfonamides, see J. G. Lombardino, J. Org. Chem., 36, 1843 (1971); for use of trifluoroacetic acid, see J. D. Catt and W. L. Matier, J. Org. Chem., 38, 1974 (1973).

Sulfonamides of Formula VI can be prepared by the reaction of sulfonyl chlorides of Formula IV with excess t-butyl amine as shown in Equation 4.

Equation4

$$JSO_2Cl \xrightarrow{\underline{t}\text{-}BuNH_2} JSO_2NH\text{-}\underline{t}\text{-}Bu$$

IV → VI

wherein

J is as previously defined.

Sulfonyl chlorides of Formula IV can be prepared according to the procedures described in U.S. 4,456,469. Alternatively, the procedures of South African Patent Application 84/8844 may be utilized which describe the conversion of mercapto or arylmethylthio compounds to sulfonyl chlorides via treatment with hypochlorite solution.

The sulfides of Formula V can be prepared by the reaction of a halo pyridine compound of Formula VII with an appropriate mercaptan in the presence of a base as described in U.S. 4,456,469 and shown in Equation 5.

Equation 5

$$J\text{-}X' \xrightarrow[\text{base}]{R'SH} J\text{-}SR'$$

VII → V

wherein

X′ is F, Cl, or Br; and

R′ is $C_1$-$C_4$ alkyl or benzyl.

A method to prepare esters of Formula VIIId is shown in Equation 6. Mercaptide displacement of a 2,6-dihalopyridine can be achieved followed by conversion to the sulfonamide VIIIa as previously described. Treatment with two equivalents of a strong base such as n-butyllithium in a similar manner to that described by P. Breant, F. Marsalis, and G. Queguiner, Synthesis, 1983, 822-824, followed by treatment with carbon dioxide affords the acids of Formula VIIIb. The acids can then be treated with appropriate alkoxides to afford the acids of Formula VIIIc which can then be esterified with the appropriate alcohols utilizing standard 1,3-dicyclohexylcarbodiimide (DCC) coupling conditions to afford esters of Formula VIIId.

Equation 6

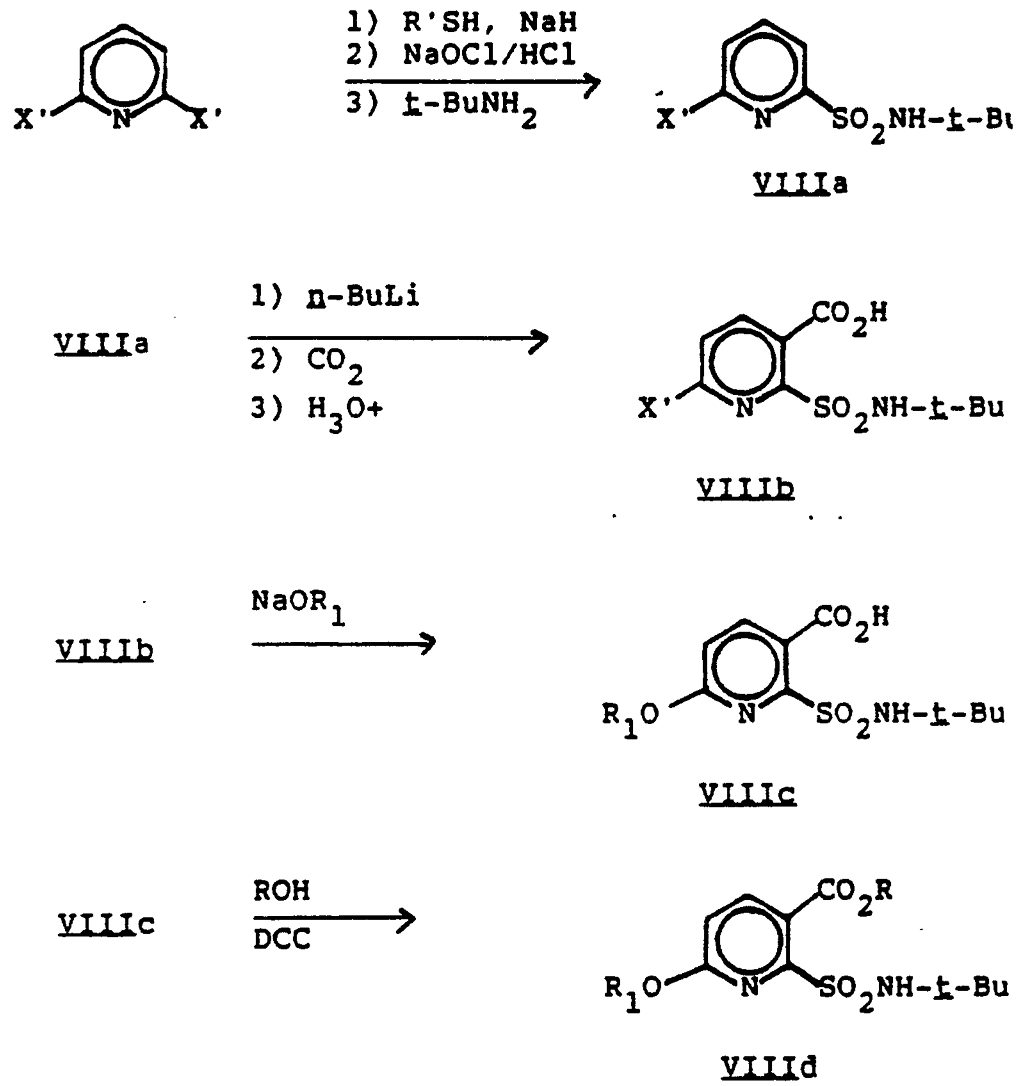


VIIIa

VIIIb

VIIIc

VIIId

wherein

X′ is F or Cl;

R′ is benzyl; and

R and $R_1$ are as previously defined.

An alternative method for Preparing esters of Formula VIIId is shown in Equation 7. The acids of Formula VIIIb can also be treated with hydroxide ion to afford compounds of Formula VIIIe which can be further reacted with appropriate alkylating reagents such as alkyl halides, in the presence of mild bases, to afford acids of Formula VIIIc which can be esterified as previously described to prepare esters of Formula VIIId.

Equation 7

VIIIb

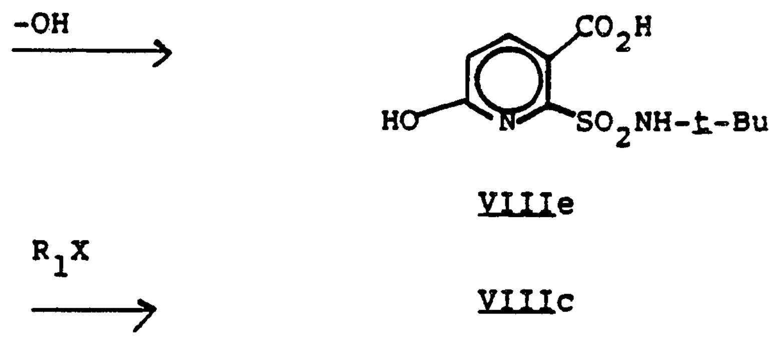


VIIIe

VIIIe → VIIIc

wherein
X is Cl, Br or I; and
$R_1$ is as previously defined.

A choice by one skilled in the art of the appropriate methods for preparing compounds of Formula I must take into account the nature of the substituents R and $R_1$, and their chemical compatibility with the reaction conditions of Equations 1 through 7.

The heterocyclic amine of Formula $A\text{-}NH_2$ in Equations 1a and 1b above can be prepared by methods known in the literature, or simple modifications thereof, by those skilled in the art. In addition, general methods for preparing aminopyrimidines have been reviewed in the following publications:

- "The Chemistry of Heterocyclic Compounds", a series published by Interscience Publishers, Inc., New York and London;
- "Pyrimidines", Vol. 16 of the same series by D.J. Brown."

Preparation in view of the above would be obvious to one skilled in the art. Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, carbonate or hydroxide). Quaternary amine salts can be made by similar techniques.

Salts of compounds of Formula I can also be prepared by exchange of one cation for another. Cationic exchange can be effected by direct contact of an aqueous solution of a salt of a compound of Formula I (e.g., alkali or quaternary amine salt) with a solution containing the cation to be exchanged. This method is most effective when the desired salt containing the exchanged cation is insoluble in water and can be separated by filtration.

Exchange may also be effected by passing an aqueous solution of a salt of a compound of Formula I (e.g., an alkali metal or quaternary amine salt) through a column packed with a cation exchange resin containing the cation to be exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I with a suitable acid, e.g., p-toluenesulfonic acid, trichloroacetic acid or the like.

The preparation of the compounds of this invention is further illustrated by the following specific examples. Temperatures are reported in degrees Celsius; abbreviations for nuclear magnetic resonance (NMR) are: s = singlet, d = doublet, t = triplet, m = multiplet, and peak positions are reported as parts per million downfield from internal tetramethylsilane. Infrared (IR) peak positions are given in reciprocal centimeters ($cm^{-1}$).

## Example 1

### Preparation of N-(1,1-dimethylethyl)-6-fluoro-2-pyridinesulfonamide

To a stirred mixture of 41 g (0.85 mol) of 50% sodium hydride (in mineral oil), which had been washed with hexanes, in 1 L dry dimethylformamide cooled to -5° C under nitrogen was added dropwise 97 mL (0.83 mol) of benzyl mercaptan. The suspension was stirred at room temperature for 1 hour, cooled to 0° C, and 100 g (0.87 mol) of 2,6-difluoropyridine was added dropwise while maintaining the temperature below 5° C. After warming to room temperature, the reaction was stirred for 20 hours. The solution was poured into ice water and the resulting solid was collected, dissolved in water/ether mixture and extracted with ether. The combined organic layers were washed with brine, dried over magnesium sulfate, and evaporated to afford 185 g tan solid. The filtrate above was also extracted with ether, washed with brine, dried over magnesium sulfate, and evaporated to afford 5 g tan solid. The 190 g (0.87 mol) of combined solids was 0 vigorously stirred in a mixture of 2.6 L methylene chloride and 1.3 L water, cooled to 0° C. To this mixture was added 338 mL (4.1 mol) concentrated hydrochloric acid followed by the dropwise addition of 5.2 L (3.5 mol) of a 5% sodium hypochlorite solution such that the temperature was maintained below 5° C. After the mixture was stirred an additional 30 minutes at 0° C, the reaction was poured into water and extracted with methylene chloride. The combined organic layers were washed with brine, dried over magnesium sulfate, and filtered. The filtrate was stirred and cooled to -70° C under nitrogen, and 318 g (4.35 mol) of t-butyl amine was added dropwise. The reaction mixture was allowed to warm to -30° C, poured into water, and extracted with methylene chloride. The combined organic layers were washed with brine, dried over magnesium sulfate, and evaporated to an oil which was purified by flash chromatography to afford a brown solid which was further washed with n-butylchloride to afford 51 g (25%) of the title compound as white solid: m.p. 94-95° C; NMR ($CDCl_3$, 200 MHz), δ 1.25 (9H, s), 5.0 (NH), 7.1 (1H, m), 7.9-8.1 (2H, m).

## Example 2

### Preparation of 2-[[(1,1-Dimethylethyl)amino]sulfonyl]-6-fluoro-3-pyridinecarboxylic acid

To a mechanically stirred solution of 50 g (0.216 mol) of the product from Example 1 in 800 mL dry tetrahydrofuran cooled to -70° C under nitrogen was added 225 mL (0.518 mol) of 2.3 molar n-butyllithium (in hexanes) such that the temperature was maintained below -65° C. The dark orange solution was stirred at -70° C for 2 hours followed by the introduction of carbon dioxide gas into the solution for 30 minutes. The suspension was warmed to -30° C, poured into water, and extracted with ether. The aqueous layer was acidified with 1 normal hydrochloric acid and extracted with methylene chloride. The combined methylene chloride layers were washed with brine, dried over magnesium sulfate, and evaporated to an oil which was triturated with n-butylchloride to afford 13.0 g (22%) of the title compound as a white solid: m.p. 152-154° C; NMR (DMSO-$d_6$, 200 MHz), δ 1.1 (9H, s), 7.55 (1H, dd, J=9 and 2Hz), 7.95 (NH), 8.3 (1H, m), 13.7 (1H, s); IR (nujol) 3200, 1730, 1470, 1335, 1145, 1125 $cm^{-1}$.

## Example 3

### Preparation of 2-[[(1,1-Dimethylethyl)amino]sulfonyl]-6-(2,2,2-trifluoroethoxy)-3-pyridinecarboxylic acid

To a stirred mixture of 3.0 g (0.062 mol) of 50% sodium hydride (in mineral oil), which had been washed with hexanes, in 60 mL dry tetrahydrofuran cooled to 0° C under nitrogen was added dropwise 4.5 mL (0.062 mol) of 2,2,2-trifluoroethanol in 25 mL dry tetrahydrofuran and stirred at room temperature for 1 hour followed by the addition of a solution of 5.6 g 0.020 mol) of the product from Example 2 in 60 mL dry tetrahydrofuran and stirred at room temperature for 20 hours. The mixture was poured into ice water and extracted with ether. The combined organic layers were washed with brine, dried over magnesium sulfate, and evaporated to a semisolid which was washed with n-butylchloride to afford 3.8 g (53%) of the title compound as a white solid: m.p. 131-133° C; NMR ($CDCl_3$, 200 MHz), δ 1.26 (9H, s), 4.9 (2H, q, J=9Hz), 7.1 (1H, d, J= 9Hz), 8.2 (1H, d, J=9Hz); IR (nujol) 3200, 1735, 1595, 1330, 1145 $cm^{-1}$.

## Example 4

Preparation of Methyl 2-[[(1,1-dimethylethyl)amino]sulfonyl]-6-(2,2,2-trifluoroethoxy)-3-pyridinecarboxylate

To a stirred solution of 4.5 g (0.0126 mol) of the product from Example 3 in 25 mL dry acetonitrile and 10 mL methanol cooled to 0°C under nitrogen was added 1.4 g (0.0176 mol) pyridine followed by the addition of 2.9 g (0.0139 mol) 1,3-dicyclohexylcarbodiimide and stirred 20 hours at room temperature. The mixture was diluted with ether and filtered. The filtrate was stripped and purified by flash chromatography to afford 3.3 g (71%) of the title compound as a white solid: m.p. 127-128°C; NMR ($CDCl_3$, 200 MHz), $\delta$ 1.28 (9H, s), 3.97 (3H, s ), 4.9 (2H, q, J=7Hz), 5.8 (NH), 7.1 (1H, d, J=8Hz), 8.1 (1H, d, J=8Hz); IR (nujol) 3320, 1715, 1600, 1325, 1170 $cm^{-1}$.

## Example 5

Preparation of Methyl 2-(aminosulfonyl)-6-(2,2,2-trifluoroethoxy)-3-pyridinecarboxylate

A solution of 3.1 g (0.0084 mol) of the product from Example 4 was stirred in 50 mL trifluoroacetic acid at room temperature for 72 hours. The solution was evaporated to a residue which was washed with ether to afford 2.4 g (92%) of the title compound as a white solid: m.p. 194-195°C; NMR (DMSO-$d_6$, 200 MHz), $\delta$3.79 (3H, s), 5.25 (2H, q, J=7Hz), 7.3 (1H, d, J=8Hz), 7.6 ($NH_2$), 8.15 (1H, d, J=8Hz); IR (nujol) 3360, 3280, 1735, 1600, 1350, 1170 $cm^{-1}$.

## Example 6

Preparation of Methyl 2-[[[[(4,6-dimethoxypyrimidin-2-yl)amino]carbonyl]amino]sulfonyl]-6-(2,2,2-trifluoroethoxy)-3-pyridinecarboxylate

To a stirred solution of 0.2 g (0.00064 mol) of the product from Example 5 and 0.28 g (0.001 mol) of phenyl (4,6-dimethoxypyrimidin-2-yl)carbamate in 3 mL acetonitrile was added dropwise 0.15 g (0.001 mol) of 1,8-diazabicyclo[5.4.0]undec-4-ene and stirred 30 minutes. The solution was diluted with water and acidified with 1 normal hydrochloric acid. The resulting precipitate was washed with water and ether to afford 0.21 g (66%) of the title compound as a white solid: m.p. 158-162°C; NMR ($CDCl_3$, 200 MHz), $\delta$ 3.95 (6H, s), 3.98 (3H, s), 4.7 (2H, q, J=7Hz), 5.8 (1H, s), 7.15 (1H, d, J=8Hz), 7.35 (NH), 8.2 (1H, d, J=8Hz), 12.8 (NH); IR (nujol) 3190, 1735, 1720, 1610, 1380, 1165 $cm^{-1}$.

By applying the procedures of Examples 1 through 6 and Equations 1 through 7, the compounds in Table I can be prepared by one skilled in the art.

TABLE I

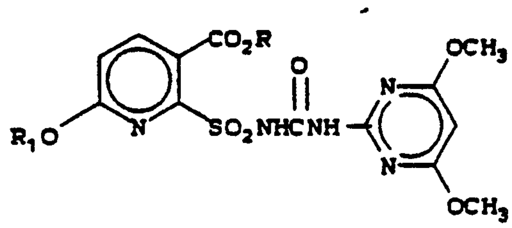

| R | $R_1$ |
|---|---|
| $CH_3$ | $CF_2H$ |
| $CH_2CH_3$ | $CF_2H$ |
| $CH_2CH_2CH_3$ | $CF_2H$ |
| $CH(CH_3)_2$ | $CF_2H$ |
| $CH_3$ | $CF_3$ |
| $CH_2CH_3$ | $CF_3$ |
| $CH_2CH_2CH_3$ | $CF_3$ |
| $CH(CH_3)_2$ | $CF_3$ |
| $CH_3$ | $OCH_2CF_2H$ |
| $CH_2CH_3$ | $OCH_2CF_2H$ |
| $CH_2CH_2CH_3$ | $OCH_2CF_2H$ |
| $CH(CH_3)_2$ | $OCH_2CF_2H$ |
| $CH_3$ | $OCH_2CF_3$ |
| $CH_2CH_3$ | $OCH_2CF_3$ |
| $CH_2CH_2CH_3$ | $OCH_2CF_3$ |
| $CH(CH_3)_2$ | $OCH_2CF_3$ |

## Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these

ingredients in the following approximate proportions:

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1963, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4; G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

## Example 7

High Strength Concentrate

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate | 99% |
| trimethylnonyl polyethylene glycol ether | 1% |

The surfactant is sprayed upon the active ingredient in a blender and the mixture sifted through a U. S. S. no. 40 sieve (0.42 mm openings) prior to packaging. The concentrate may be formulated further for practical use.

## Example 8

Wettable Powder

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate | 65% |
| dodecylphenol polyethylene glycol ether | 2% |
| sodium ligninsulfonate | 4% |
| sodium silicoaluminate | 6% |
| montmorillonite (calcined) | 23% |

The ingredients are thoroughly blended. The liquid surfactant is added by spraying upon the solid ingredients in the blender. After grinding in a hammer mill to produce particles essentially all below 100 microns, the material is reblended and sifted through a U.S.S. No. 50 sieve (0.3 mm opening) and packaged.

## Example 9

Aqueous Suspension

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate | 50.0% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

## Example 10

Oil Suspension

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate | 35% |
| blend of polyalcohol carboxylic esters and oil soluble petroleum sulfonates | 6% |
| xylene | 59% |

The ingredients are combined and ground together in a sand mill to produce particles essentially all below 3 microns. The product can be used directly, extended with oils, or emulsified in water.

Example 11

Oil Suspension

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied to directly, but preferably after being extended with oils or emulsified in water.

## Example 12

### Aqueous Suspension

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate | 25% |
| hydrated attapulgite | 3% |
| crude calcium ligninsulfonate | 10% |
| sodium dihydrogen phosphate | 0.5% |
| water | 61.5% |

The ingredients are ground together in a ball or roller mill until the solid particles have been reduced to diameters under 10 microns.

Example 13

Wettable Powder

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate | 40.0% |
| dioctyl sodium sulfosuccinate | 1.5% |
| sodium ligninsulfonate | 3% |
| low viscosity methyl cellulose | 1.5% |
| attapulgite | 54% |

The ingredients are thoroughly blended, passed through an air mill, to produce an average particle size under 15 microns, reblended, and sifted through a U.S.S. No. 50 sieve (0.3 mm opening) before packaging.

All compounds of the invention may be formulated in the same manner.

## Example 14

### Granule

| | |
|---|---|
| wettable powder of Example 13 | 15% |
| gypsum | 69% |
| potassium sulfate | 16% |

The ingredients are blended in a rotating mixer and water sprayed on to accomplish granulation. When most of the material has reached the desired range of 1.0 to 0.42 cm (U.S.S.#18 to 40 sieves), the granules are removed, dried, and screened. Oversized material is crushed to produce additional material in the desired range. These granules contain % active ingredient.

## Example 15

### Wettable Powder

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate | 50% |
| sodium alkylnaphthalenesulfonate | 2% |
| low viscosity methyl cellulose | 2% |
| diatomaceous earth | 46% |

The ingredients are blended, coarsely hammer-milled and the air milled to produce particles of active essentially all below 10 microns in diameter. The product is reblended before packaging.

## Example 16

### Extruded Pellet

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate | 25% |
| anhydrous sodium sulfate | 10% |
| crude calcium ligninsulfonate | 5% |
| sodium alkylnaphthalenesulfonate | 1% |
| calcium/magnesim bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

## Example 17

### Wettable Powder

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate | 80% |
| sodium alkylnaphthalenesulfonate | 2% |
| sodium ligninsulfonate | 2% |
| synthetic amorphous silica | 3% |
| kaolinite | 13% |

The ingredients are blended and then ground in a hammermill to produce particles with an average particle size less than 25 microns in diameter. The material is reblended and sifted through a U.S.S. No. 50 sieve (0.3 mm opening) before being packaged.

Example 18

High Strength Concentrate

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate | 98.5% |
| silica aerogel | 0.5% |
| synthetic amorphous fine silica | 1.0% |

The ingredients are blended and ground in a hammer mill to produce a high strength concentrate essentially all passing a U.S.S. No. 50 sieve (0.3 mm openings). This material may then be formulated in a variety of ways.

## Example 19

### High Strength Concentrate

| | |
|---|---|
| methyl 2-[[[[(4,6-dimethoxypyrimidin-2-yl)amino]carbonyl]amino]sulfonyl]-6-(2,2,2-trifluoroethoxy)-3-pyridinecarboxylate, sodium salt | 5% |
| water | 95% |

The salt is added directly to the water with stirring to produce the solution, which may then be packaged for use.

## UTILITY

Test results indicate that the compounds pertaining to the present invention are highly active preemergent and/or postemergent herbicides or plant growth regulants. These compounds have utility for pre- and/or postemergence broad-spectrum control of broadleaf and grass weeds, especially blackgrass. As many of these compounds are relatively safe to cereal crops, such as wheat, barley, oats, rye and triticale, they are particularly useful for control of blackgrass in these crops. These compounds also have utility for broad-spectrum pre- and/or postemergence weed control in areas where complete control of all vegetation is desired such as around storage tanks, parking lots, drive-in theaters, billboards, highways, railroad structures, and in fallow areas. Alternatively, these compounds are useful to modify plant growth.

Rates of application for compounds of this invention are determined by a number of factors. These factors include: formulation selected, method of application, amount of vegetation present, growing conditions, etc. In general terms, the subject compounds should be applied at rates of from 0.001 to 20 kg/ha, with a preferred range of from 0.004 to 0.25 kg/ha. Compounds of this invention may be used alone or in combination with other commercial herbicides, insecticides, or fungicides. The following list exemplifies some of the herbicides suitable for use in mixtures. A combination of a compound from this invention with one or more of the following herbicides may be particularly useful for weed control.

| Common Name | Chemical Name |
|---|---|
| barban | 4-chloro-2-butynyl 3-chlorocarbamate |
| bensulfuron methyl | 2-[[[[[(4,6-dimethoxy-2-pyrimidinyl)amino]carbonyl]-amino]sulfonyl]methyl]benzoic acid, methyl ester |
| bentazon | 3-(1-methylethyl)-(1H)-2,1,3-benzothiadiazin-4(3H)-one, 2,2-dioxide |
| benzoylprop | N-benzoyl-N-(3,4-dichlorophenyl)-DL-alanine |
| bifenox | methyl 5-(2,4-dichlorophenoxy)-2-nitrobenzoate |
| bromoxynil | 3,5-dibromo-4-hydroxybenzonitrile |
| butachlor | N-(butoxymethyl)-2-chloro-N-(2,6-diethylphenyl)acetamide |
| chlorpropham | 1-methylethyl 3-chlorophenylcarbamate |
| chlorsulfuron | 2-chloro-N-[[(4-methoxy-6-methyl-1,3,5-triazin-2-yl)amino]-carbonyl]benzenesulfonamide |
| chlortoluron | N'-(3-chloro-4-methylphenyl)-N,N-dimethylurea |
| cinmethylin | exo-1-methyl-4-(1-methylethyl)-2-[(2-methylphenyl)methoxy]-7-oxabicyclo[2.2.1]heptane |
| DCPA | dimethyl 2,3,5,6-tetrachloro-1,4-benzenedicarboxylate |
| diallate | S-(2,3-dichloro-2-propenyl)bis(1-methylethyl)carbamothioate |
| dicamba | 3,6-dichloro-2-methoxybenzoic acid |

| Common Name | Chemical Name |
|---|---|
| dichlobenil | 2,6-dichlorobenzonitrile |
| dichlorprop | (±)-2-(2,4-dichlorophenoxy)propanoic acid |
| dichlofop | (±)-2-[4-(2,4-dichlorophenoxy)-phenoxy]propanoic acid |
| difenzoquat | 1,2-dimethyl-3,5-diphenyl-1H-pyrazolium |
| DNOC | 2-methyl-4,6-dinitrophenol |
| DPX-M6316 | 3-[[[[(4-methoxy-6-methyl-1,3,5-triazin-2-yl)amino]carbonyl]-amino]sulfonyl]-2-thiophene-carboxylic acid, methyl ester |
| DPX-L5300 | 2-[[[[N-(4-methoxy-6-methyl-1,3,5-triazine-2-yl)-N-methylamino]-carbonyl]amino]sulfonyl]benzoic acid, methyl ester |
| flamprop | N-benzoyl-N-(3-chloro-4-fluoro-phenyl)-DL-alanine |
| fluazifop | (±)-2-[4-[[5-(trifluoromethyl)-2-pyridinyl]oxy]phenoxy]propanoic acid |
| fluazifop-P | (R)-2-[4-[[5-(trifluoromethyl)-2-pyridinyl]oxy]phenoxy]propanoic acid |
| fluchloralin | N-(2-chloroethyl)-2,6-dinitro-N-propyl-4-(trifluoromethyl)-benzenamine |
| fluorochlor-idone | 3-chloro-4-(chloromethyl)-1-[3-(tri-fluoromethyl)phenyl]-2-pyrrolidinone |
| imazametha-benz | 6-(4-isopropyl-4-methyl-5-oxo-2-imidazolin-2-yl)-m-toluic acid, methyl ester and 6-(4-isopropyl-4-methyl-5-oxo-2-imidazolin-2-yl)-p-toluic acid, methyl ester |

| Common Name | Chemical Name |
|---|---|
| ioxynil | 4-hydroxy-3,5-diiodobenzonitrile |
| isoproturon | N-(4-isopropylphenyl)-N',N'-dimethylurea |
| linuron | N'-(3,4-dichlorophenyl)-N-methoxy-N-methylurea |
| MCPA | (4-chloro-2-methylphenoxy)acetic acid |
| MCPB | 4-(4-chloro-2-methylphenoxy)butanoic acid |
| mecoprop | (±)-2-(4-chloro-2-methylphenoxy)-propanoic acid |
| mefluidide | N-[2,4-dimethyl-5-[[(trifluoro-methyl)sulfonyl]amino]-phenyl]acetamide |
| methabenz-thiazuron | 1,3-dimethyl-3-(2-benzothiazolyl)-urea |
| methoxuron | N'-(3-chloro-4-methoxyphenyl)-N,N-dimethylurea |
| metribuzin | 4-amino-6-(1,1-dimethylethyl)-3-(methylthio)-1,2,4-triazin-5(4H)-one |
| metsulfuron methyl | 2-[[[[(4-methoxy-6-methyl-1,3,5-tri-azin-2-yl)amino]carbonyl]-amino]sulfonyl]benzoic acid, methyl ester |
| monuron | N'-(4-chlorophenyl)-N,N-dimethylurea |
| neburon | 1-butyl-3-(3,4-dichlorophenyl)-1-methyl urea |
| picloram | 4-amino-3,5,6-trichloro-2-pyridine-carboxylic acid |
| PPG-1013 | 5-[2-chloro-4-(trifluoromethyl)-phenoxy]-2-nitroacetophenone oxime-O-acetic acid, methyl ester |

| Common Name | Chemical Name |
|---|---|
| propanil | N-(3,4-dichlorophenyl)propanamide |
| sulfometuron methyl | 2-[[[[(4,6-dimethyl-2-pyrimidinyl)-amino]carbonyl]amino]sulfonyl]-benzoic acid, methyl ester |
| terbuthyl-azine | 2-(tert-butylamino)-4-chloro-6-(ethylamino)-s-triazine |
| terbutryn | N-(1,1-dimethylethyl)-N'-ethyl-6-(methylthio)-1,3,5-triazine-2,4-diamine |
| triallate | S-(2,3,3-trichloro-2-propenyl) bis(1-methylethyl)carbamothioate |
| trifluralin | 2,6-dinitro-N,N-dipropyl-4-(tri-fluoromethyl)benzenamine |
| 2,4-D | (2,4-dichlorophenoxy)acetic acid |
| 2,4-DB | 4-(2,4-dichlorophenoxy)butanoic acid |

The selective herbicidal properties of the subject compounds that make them useful as cereal herbicides were discovered in a number of greenhouse tests. Test descriptions and results follow.

COMPOUNDS

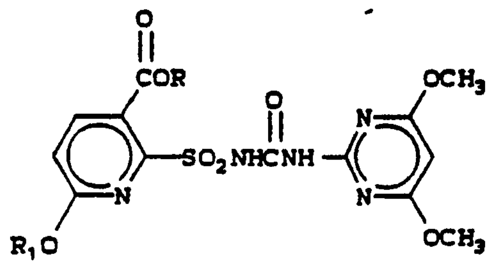

| COMPOUND | R | $R_1$ | m.p. (°C) |
|---|---|---|---|
| 1 | $CH_3$ | $CH_2CF_3$ | 158-162 |
| 2 | $CH_2CH_3$ | $CH_2CF_3$ | 173-175 |
| 3 | $CH_2CH_2CH_3$ | $CH_2CF_3$ | 138-141 |
| 4 | $CH_3$ | $CHF_2$ | 174-175 |
| 5 | $CH_2CH_3$ | $CHF_2$ | 159-163 |

TEST A

Seeds of barley (*Hordeum vulgare*), barnyardgrass (*Echinochloa crus-galli*), cheat grass (*Bromus secalinus*), cocklebur (*Xanthium pensylvanicum*), corn (*Zea mays*), cotton (*Gossypium hirsutum*), crabgrass (*Digitaria* spp.), giant foxtail (*Setaria faberi*), morningglory (*Ipomoea* spp.), rice (*Oryza sativa*), sorghum (*Sorghum bicolor*), soybean (*Glycine max*), sugar beet (*Beta vulgaris*), velvetleaf (*Abutilon theophrasti*), wheat (*Triticum aestivum*), and wild oat (*Avena fatua*) and nutsedge (*Cyperus rotundus*) tubers were planted and treated preemergence with test chemicals dissolved in as non-phytotoxic solvent. At the same time, these crop and weed species were also treated with postemergence applications of test chemicals. Plants ranged in height from two to eighteen cm (two to three leaf stage) for postemergence treatments. Treated plants and controls were maintained in a greenhouse for sixteen days, after which all species were compared to controls and visually evaluated. The ratings, summarized in Table A, are based on a scale of 0 to 10 where 0 is no effect and 10 is complete control. A dash (-) response means no test result. The accompanying descriptive symbols have the following meanings:

C = chlorosis/necrosis;
E = inhibition of emergence;
G = growth retardation; and
H = hormonal or formative effect.

TABLE A

| | CMPD 1 | | CMPD 2 | |
|---|---|---|---|---|
| Rate (g/ha) | 50 | 10 | 50 | 10 |
| POSTEMERGENCE | | | | |
| Cotton | 9C | 10C | 5C,9G | 8G |
| Morningglory | 10C | 4C,9G | 10C | 9C |
| Cocklebur | 10C | 10C | 9C | 9C |
| Nutsedge | 0 | 0 | 0 | 0 |
| Crabgrass | 2G | 2G | 3G | 0 |
| Barnyardgrass | 3C,9H | 3C,9H | 3C,8H | 3C,7G |
| Wild oat | 0 | 0 | 0 | 0 |
| Wheat | 5G | 3G | 3G | 0 |
| Corn | 3C,9G | 3C,7H | 2C,9G | 2C,7G |
| Soybean | 5C,9G | 5C,9G | 4C,9H | 2C,9G |
| Rice | 3C,8G | 2C,8G | 2C,9G | 3C,6G |
| Sorghum | 3C,8H | 3C,8H | 2C,6G | 4C |
| Cheat grass | 2C,7G | 7G | - | - |
| Sugar beet | 10C | 9C | 4C,9G | 9C |
| Velvetleaf | 10C | 3C,9G | 3C,8G | 2C,5G |
| Giant foxtail | 6G | 4G | 0 | 0 |
| Barley | 6G | 2G | 2C,7G | 2G |

TABLE A

| | CMPD 1 | | CMPD 2 | |
|---|---|---|---|---|
| Rate (g/ha) | 50 | 10 | 50 | 10 |
| PREEMERGENCE | | | | |
| Cotton | 9G | 8G | 3G | - |
| Morningglory | 9G | 2C,7G | 8G | 2C,7H |
| Cocklebur | 4C,8H | 3C,7H | 8H | 3C,5H |
| Nutsedge | 0 | 0 | 10E | 0 |
| Crabgrass | 0 | 0 | 0 | 0 |
| Barnyardgrass | 2C,7H | 2G | 3G | 0 |
| Wild oat | 0 | 0 | 0 | 0 |
| Wheat | 2G | 0 | 4G | 0 |
| Corn | 3C,7H | 2G | 3C,7G | 2C,5G |
| Soybean | 4C,8H | 3C,7H | 3C,8H | 3C,3H |
| Rice | 8G | 2G | 8G | 3G |
| Sorghum | 8G | 2G | 5G | 0 |
| Cheat grass | 8G | 2G | - | - |
| Sugar beet | 3C,9G | 8G | 6G | 3G |
| Velvetleaf | 3C,8G | 3G | 1C,1H | 0 |
| Giant foxtail | 2G | 0 | 0 | 0 |
| Barley | 7G | 5G | 5G | 3G |

TABLE A

| | CMPD 3 | | CMPD 4 | |
|---|---|---|---|---|
| Rate (g/ha) | 50 | 10 | 50 | 10 |
| POSTEMERGENCE | | | | |
| Cotton | 5C,9G | 2C,7G | 10C | 5C,9G |
| Morningglory | 4C,9G | 4C,9G | 5C,9G | 2C,5H |
| Cocklebur | 5C,9G | 3C,8H | 10C | 9C |
| Nutsedge | 5G | 0 | 5C,9G | 10C |
| Crabgrass | 3G | 0 | 2G | 2G |
| Barnyardgrass | 3C,8H | 3C,7G | 5C,9G | 3C,9H |
| Wild oat | 0 | 0 | 3G | 0 |
| Wheat | 0 | 0 | 0 | 0 |
| Corn | 2C,5G | 3G | 3C,8G | 7G |
| Soybean | 4C,8H | 3C,7H | 5C,9G | 4C,9G |
| Rice | 4C,7G | 3C,5G | 3C,9G | 8G |
| Sorghum | 4C,8G | 5G | 5G | 0 |
| Cheat grass | 3G | 0 | 0 | 4G |
| Sugar beet | 3C,7G | 4G | 9C | 9C |
| Velvetleaf | 7G | 3G | 10C | 8G |
| Giant foxtail | 4G | 2C,3G | 4G | 4G |
| Barley | 0 | 0 | 7G | 5G |

TABLE A

| | CMPD 3 | | CMPD 4 | |
|---|---|---|---|---|
| Rate (g/ha) | 50 | 10 | 50 | 10 |
| PREEMERGENCE | | | | |
| Cotton | 3H | 0 | 9G | 6G |
| Morningglory | 8G | 3H | 7G | 6G |
| Cocklebur | 3C,6H | 2C,5G | 9H | 3C,7H |
| Nutsedge | 5G | 0 | 8G | 7G |
| Crabgrass | 5G | 0 | 3G | 0 |
| Barnyardgrass | 3C,7G | 0 | 7G | 2H |
| Wild oat | 0 | 0 | 2G | 0 |
| Wheat | 0 | 0 | 0 | 0 |
| Corn | 3C,8H | 2C,3G | 3C,5G | 2G |
| Soybean | 3C,3H | 2G | 4C,9H | 3C,7G |
| Rice | 3C,7G | 2C,5G | 9H | 6G |
| Sorghum | 3C,7G | 2C,2G | 2G | 0 |
| Cheat grass | 3G | 0 | 4G | 0 |
| Sugar beet | 7G | 6G | 3C,9G | 9G |
| Velvetleaf | 2H | 0 | 9G | 7G |
| Giant foxtail | 5G | 0 | 2G | 0 |
| Barley | 2C,3G | 0 | 8G | 7G |

TABLE A

| | CMPD 5 | |
|---|---|---|
| Rate (g/ha) | 50 | 10 |
| POSTEMERGENCE | | |
| Cotton | 9C | 10C |
| Morningglory | 3C,7G | 2C,6G |
| Cocklebur | 10C | 9C |
| Nutsedge | 9C | 4C,9G |
| Crabgrass | 2G | 0 |
| Barnyardgrass | 5C,9G | 9H |
| Wild oat | 0 | 0 |
| Wheat | 2G | 0 |
| Corn | 3C,8H | 2C,4H |
| Soybean | 9C | 5C,9G |
| Rice | 9C | 7G |
| Sorghum | 2C,3G | 1C |
| Cheat grass | 7G | 7G |
| Sugar beet | 5C,9G | 3C,7G |
| Velvetleaf | 2C,8G | 3G |
| Giant foxtail | 3C,5G | 3C,3G |
| Barley | 6G | 0 |

TABLE A

| | CMPD 5 | |
|---|---|---|
| Rate (g/ha) | 50 | 10 |
| PREEMERGENCE | | |
| Cotton | 9G | 8G |
| Morningglory | 8G | 7G |
| Cocklebur | 9H | 9H |
| Nutsedge | 8G | 0 |
| Crabgrass | 4H | 3G |
| Barnyardgrass | 8H | 2C,5G |
| Wild oat | 2C,2G | 0 |
| Wheat | 0 | 0 |
| Corn | 3C,7G | 3C,6G |
| Soybean | 4C,9H | 3C,7H |
| Rice | 3C,9H | 3C,8G |
| Sorghum | 0 | 2C,2G |
| Cheat grass | 2G | 0 |
| Sugar beet | 8G | 6G |
| Velvetleaf | 5H | 3H |
| Giant foxtail | 5G | 0 |
| Barley | 3C,7G | 3G |

<u>TEST B</u>

Seeds of barley (*Hordeum vulgare*), barnyardgrass (*Echinochloa crus-galli*), blackgrass (*Alopecurus*

*myosuroides*), chickweed (*Stellaria media*), cocklebur (*Xanthium pensylvanicum*), corn (*Zea mays*), cotton (*Gossypium hirsutum*), crabgrass (*Digitaria* spp.), downy brome (*Bromus tectorum*), giant foxtail (*Setaria faberi*), green foxtail (*Setaria viridis*), jimsonweed (*Datura stramonium*), johnsongrass (*Sorghum halepense*), lambsquarters (*Chenopodium album*), morningglory (*Ipomoea* spp.), rape (*Brassica napus*), rice (*Oryza sativa*), sicklepod (*Cassia obtusifolia*), soybean (*Glycine max*), sugar beet (*Beta vulgaris*), teaweed (*Sida spinosa*), velvetleaf (*Abutilon theophrasti*), wheat (*Triticum aestivum*), wild buckwheat (*Polygonum convolvulus*), and wild oat (*Avena fatua*) and nutsedge (*Cyperus rotundus*) tubers were planted and treated preemergence with test chemicals dissolved in a non-phytotoxic solvent. At the same time, these crop and weed species were also treated with postemergence applications of test chemicals. Plants ranged in height from two to eighteen cm (two to three leaf stage) for postemergence treatments. Treat plants and controls were maintained in a greenhouse for approximately 24 days, after which all species were compared to controls and visually evaluated. The ratings, summarized in Table B, are based on a scale of 0 to 100 where 0 is no effect and 100 is complete control. A dash (-) response means no test result.

TABLE B

CMPD 1

| Rate (g/ha) | 62 | 16 | 4 | 1 |
|---|---|---|---|---|
| POSTEMERGENCE | | | | |
| Giant Foxtail | 0 | 0 | 0 | 0 |
| Velvetleaf | 100 | 100 | 70 | 50 |
| Sugar beet | 100 | 100 | 100 | 90 |
| Crabgrass | 50 | 30 | 0 | 0 |
| Teaweed | 70 | 60 | 50 | 30 |
| Jimsonweed | 100 | 100 | 100 | 90 |
| Rice | 70 | 50 | 30 | 0 |
| Cocklebur | 100 | 100 | 70 | 50 |
| Cotton | 100 | 90 | 70 | 30 |
| Soybean | 100 | 100 | 90 | 80 |
| Barnyardgrass | 60 | 50 | 30 | 0 |
| Wild oat | 30 | 0 | 0 | 0 |
| Morningglory | 90 | 70 | 60 | 50 |
| Wheat | 0 | 0 | 0 | 0 |
| Sicklepod | 100 | 100 | 100 | 70 |
| Johnsongrass | 60 | 30 | 0 | 0 |
| Nutsedge | 60 | 50 | 40 | 30 |
| Corn | 0 | 0 | 0 | 0 |
| Wild buckwheat | 100 | 90 | 80 | 70 |
| Blackgrass | 100 | 100 | 100 | 100 |
| Rape | 100 | 100 | 100 | 100 |
| Barley | 50 | 30 | 0 | 0 |
| Green foxtail | 40 | 30 | 0 | 0 |
| Lambsquarters | 100 | 100 | 90 | 70 |
| Chickweed | 100 | 80 | 70 | 50 |
| Downy brome | 90 | 80 | 70 | 60 |

TABLE B

CMPD 1

| Rate (g/ha) | 250 | 62 | 16 | 4 |
|---|---|---|---|---|
| PREEMERGENCE | | | | |
| Giant Foxtail | 90 | 30 | 0 | 0 |
| Velvetleaf | 100 | 90 | 70 | 30 |
| Sugar beet | 100 | 100 | 90 | 80 |
| Crabgrass | 90 | 70 | 30 | 0 |
| Teaweed | 100 | 95 | 90 | 85 |
| Jimsonweed | 100 | 100 | 90 | 50 |
| Rice | 100 | 100 | 90 | 70 |
| Cocklebur | 100 | 100 | 90 | 30 |
| Cotton | 100 | 90 | 60 | 30 |
| Soybean | 100 | 100 | 90 | 60 |
| Barnyardgrass | 100 | 100 | 80 | 50 |
| Wild oat | 60 | 30 | 0 | 0 |
| Morningglory | 100 | 100 | 90 | 60 |
| Wheat | 70 | 50 | 0 | 0 |
| Sicklepod | 100 | 100 | 90 | 70 |
| Johnsongrass | 90 | 80 | 30 | 0 |
| Nutsedge | 100 | 60 | 30 | 0 |
| Corn | 100 | 90 | 50 | 0 |
| Wild buckwheat | 100 | 90 | 80 | 70 |
| Blackgrass | 100 | 90 | 60 | 30 |
| Rape | 100 | 100 | 100 | 90 |
| Barley | 90 | 60 | 30 | 0 |
| Green foxtail | 90 | 70 | 30 | 0 |
| Lambsquarters | 100 | 100 | 100 | 90 |
| Chickweed | 90 | 80 | 70 | 50 |
| Downy brome | 100 | 80 | 50 | 30 |

TABLE B

CMPD 2

| Rate (g/ha) | 250 | 62 | 16 | 4 |
|---|---|---|---|---|
| POSTEMERGENCE | | | | |
| Giant Foxtail | 50 | 30 | 0 | 0 |
| Velvetleaf | 90 | 80 | 60 | 30 |
| Sugar beet | 100 | 100 | 100 | 60 |
| Crabgrass | 50 | 30 | 0 | 0 |
| Teaweed | 70 | 60 | 50 | 30 |
| Jimsonweed | 100 | 100 | 100 | 90 |
| Rice | 70 | 50 | 30 | 0 |
| Cocklebur | 100 | 100 | 100 | 90 |
| Cotton | 100 | 70 | 50 | 30 |
| Soybean | 100 | 100 | 100 | 90 |
| Barnyardgrass | 90 | 70 | 50 | 30 |
| Wild oat | 0 | 0 | 0 | 0 |
| Morningglory | 100 | 90 | 80 | 70 |
| Wheat | 30 | 0 | 0 | 0 |
| Sicklepod | 100 | 90 | 70 | 60 |
| Johnsongrass | 70 | 60 | 50 | 30 |
| Nutsedge | 100 | 30 | 0 | 0 |
| Corn | 90 | 70 | 50 | 30 |
| Wild buckwheat | 100 | 100 | 90 | 60 |
| Blackgrass | 100 | 100 | 70 | 50 |
| Rape | 100 | 100 | 100 | 100 |
| Barley | 50 | 30 | 0 | 0 |
| Green foxtail | 60 | 30 | 0 | 0 |
| Lambsquarters | 70 | 50 | 0 | 0 |
| Chickweed | 100 | 90 | 70 | 50 |
| Downy brome | 90 | 60 | 30 | 0 |

TABLE B

CMPD 2

| Rate (g/ha) | 250 | 62 | 16 |
|---|---|---|---|
| PREEMERGENCE | | | |
| Giant Foxtail | 50 | 30 | 0 |
| Velvetleaf | 80 | 50 | 30 |
| Sugar beet | 100 | 90 | 70 |
| Crabgrass | 50 | 30 | 0 |
| Teaweed | 70 | 50 | 30 |
| Jimsonweed | 90 | 80 | 50 |
| Rice | 100 | 90 | 80 |
| Cocklebur | 90 | 70 | 50 |
| Cotton | 60 | 30 | - |
| Soybean | 50 | 30 | 0 |
| Barnyardgrass | 90 | 60 | 0 |
| Wild oat | 0 | 0 | 0 |
| Morningglory | 90 | 80 | 60 |
| Wheat | 50 | 0 | 0 |
| Sicklepod | 90 | 60 | 30 |
| Johnsongrass | 90 | 70 | 30 |
| Nutsedge | 70 | 50 | 30 |
| Corn | 60 | 0 | 0 |
| Wild buckwheat | 90 | 80 | 70 |
| Blackgrass | 90 | 80 | 70 |
| Rape | 100 | 90 | 80 |
| Barley | 30 | 0 | 0 |
| Green foxtail | 60 | 30 | 0 |
| Lambsquarters | 100 | 100 | 90 |
| Chickweed | 100 | 90 | 70 |
| Downy brome | 80 | 50 | 30 |

## TEST C

Seeds of annual bluegrass (*Poa annua*), spring and winter barley (*Hordeum vulgare*), blackgrass (*Alopecurus myosuroides*), black nightshade (*Solanum nigrum*), catchweed bedstraw (*Galium aparine*), cheat grass (*Bromus secalinus*), downy brome (*Bromus tectorum*), field pennycress (*Thlaspi arvense*), field violet (*Viola arvensis*), green foxtail (*Setaria viridis*), Italian ryegrass (*Lolium multiflorum*), ivyleaf speedwell (*Veronica hederaefolia*), jointed goatgrass (*Aegilops cylindrica*), kochia (*Kochia scoparia*), lambsquarters (*Chenopodium album*), Persian speedwell (*Veronica persica*), rape (*Brassica napus*), Russian thistle (*Salsola kali*), scentless chamomile (*Matricaria inodora*), sugar beet (*Beta vulgaris*), spring and winter wheat (*Triticum aestivum*), wild buckwheat (*Polygonum convolvulus*), and wild oat (*Avena fatua*) were planted and treated preemergence with test chemicals dissolved in a non-phytotoxic solvent. At the same time, these crop and weed species were also treated with postemergence applications of test chemicals. Plants ranged in height from two to twenty-four cm (two to three leaf stage) for postemergence treatments. Blackgrass and wild oat were treated postemergence at two growth stages -- the first stage being at two to three leaves and the second stage being approximately at four leaves or in the initial stages of tillering. Treated plants and controls were maintained in a greenhouse for approximately 21 days, after which all species were compared to controls and visually evaluated. The ratings, summarized in Table C, are based on a scale of 0 to 100 where 0 is no effect and 100 is complete control. A dash (-) response means no test result.

TABLE C

CMPD 1

| Rate (g/ha) | 125 | 64 | 32 | 16 | 8 | 4 | 2 | 1 |
|---|---|---|---|---|---|---|---|---|
| POSTEMERGENCE | | | | | | | | |
| Spring wheat | 20 | 10 | 0 | 0 | 0 | 0 | 0 | 0 |
| Spring barley | 40 | 40 | 20 | 20 | 0 | 0 | 0 | 0 |
| Wild oat | 20 | 0 | 0 | 0 | 0 | 0 | 0 | 0 |
| Downy brome | 0 | 0 | 0 | 0 | 0 | 0 | 0 | 0 |
| Cheat grass | 60 | 40 | 40 | 10 | 0 | 0 | 0 | 0 |
| Blackgrass | 90 | 90 | 90 | 80 | 60 | 50 | 30 | 0 |
| Ann. bluegrass | 30 | 10 | 0 | 0 | 0 | 0 | 0 | 0 |
| Green foxtail | 10 | 0 | 0 | 0 | 0 | 0 | 0 | 0 |
| Italian ryegrass | 70 | 60 | 30 | 10 | 0 | 0 | 0 | 0 |
| Rape | 100 | 100 | 100 | 100 | 100 | 100 | 90 | 70 |
| Winter wheat | 20 | 10 | 0 | 0 | 0 | 0 | 0 | 0 |
| Winter barley | 40 | 30 | 10 | 10 | 0 | 0 | 0 | 0 |
| Jointed goatgrass | 20 | 20 | 10 | 0 | 0 | 0 | 0 | 0 |
| Wild oat (Stg2) | 30 | 20 | 0 | 0 | 0 | 0 | 0 | 0 |
| Blackgrass (Stg2) | 100 | 90 | 80 | 70 | 60 | 50 | 20 | 0 |
| Ctchwd. bedstraw | 90 | 70 | 70 | 50 | 30 | 0 | 0 | 0 |
| Wild buckwheat | 100 | 90 | 70 | 60 | 50 | 40 | 10 | 0 |
| Kochia | 60 | 40 | 30 | 20 | 10 | 0 | 0 | 0 |
| Sntls. chamomile | 100 | 100 | 100 | 90 | 80 | 60 | 30 | 0 |
| Blk. nightshade | 100 | 70 | 70 | 50 | 20 | 20 | 10 | 0 |
| Russian thistle | 70 | 50 | 50 | 0 | 0 | 0 | 0 | 0 |
| Persn. speedweed | 10 | 0 | 0 | 0 | 0 | 0 | 0 | 0 |
| Sugarbeet | 100 | 100 | 100 | 100 | 100 | 100 | 90 | 80 |
| Ivylf. speedwell | 100 | 100 | 90 | 80 | 80 | 70 | 50 | 10 |
| Lambsquarters | 50 | 50 | 40 | 20 | 10 | 10 | 0 | 0 |
| Pennycress | 100 | 100 | 90 | 90 | 70 | 50 | 40 | 0 |
| Field violet | 90 | 80 | 50 | 50 | 20 | 10 | 0 | 0 |

TABLE C

CMPD 1

| Rate (g/ha) | 125 | 64 | 32 | 16 | 8 | 4 | 2 | 1 |
|---|---|---|---|---|---|---|---|---|
| PREEMERGENCE | | | | | | | | |
| Spring wheat | 50 | 50 | 10 | 0 | 0 | 0 | 0 | 0 |
| Spring barley | 80 | 70 | 40 | 20 | 0 | 0 | 0 | 0 |
| Wild oat | 20 | 0 | 0 | 0 | 0 | 0 | 0 | 0 |
| Downy brome | 80 | 50 | 0 | 0 | 0 | 0 | 0 | 0 |
| Cheat grass | 80 | 70 | 20 | 0 | 0 | 0 | 0 | 0 |
| Blackgrass | 100 | 100 | 90 | 60 | 50 | 20 | 10 | 0 |
| Ann. bluegrass | 70 | 70 | 50 | 30 | 10 | 0 | 0 | 0 |
| Green foxtail | 60 | 60 | 0 | 0 | 0 | 0 | 0 | 0 |
| Italian ryegrass | 80 | 80 | 70 | 40 | 20 | 0 | 0 | 0 |
| Rape | 100 | 100 | 100 | 100 | 100 | 100 | 40 | 0 |
| Winter wheat | 20 | 10 | 10 | 0 | 0 | 0 | 0 | 0 |
| Winter barley | 80 | 70 | 50 | 10 | 0 | 0 | 0 | 0 |
| Jointed goatgrass | 20 | 10 | 0 | 0 | 0 | 0 | 0 | 0 |
| Ctchwd. bedstraw | 100 | 100 | 80 | 70 | 40 | 20 | 20 | 20 |
| Wild buckwheat | 100 | 100 | 90 | 70 | 50 | 40 | 10 | 0 |
| Kochia | 70 | 50 | 50 | 20 | 0 | 0 | 0 | 0 |
| Sntls. chamomile | 100 | 100 | 90 | 90 | 80 | 70 | 10 | 0 |
| Blk. nightshade | 80 | 70 | 60 | 60 | 30 | 20 | 20 | 0 |
| Russian thistle | 90 | 50 | 40 | 40 | 20 | 0 | 0 | 0 |
| Persn. speedweed | 100 | 80 | 80 | 60 | 40 | 40 | 30 | 0 |
| Sugarbeet | 100 | 100 | 100 | 100 | 90 | 80 | 80 | 80 |
| Ivylf. speedwell | 100 | 90 | 90 | 70 | 90 | 80 | 60 | 20 |
| Lambsquarters | 90 | 90 | 80 | 70 | 30 | 0 | 0 | 0 |
| Pennycress | 100 | 90 | 90 | 90 | 80 | 80 | 60 | 50 |
| Field violet | 100 | 100 | 100 | 90 | 80 | 70 | 60 | 60 |

TABLE C

CMPD 2

| Rate (g/ha) | 125 | 64 | 32 | 16 | 8 | 4 |
|---|---|---|---|---|---|---|
| POSTEMERGENCE | | | | | | |
| Spring wheat | 0 | 0 | 0 | 0 | 0 | 0 |
| Spring barley | 20 | 10 | 0 | 0 | 0 | 0 |
| Wild oat | 0 | 0 | 0 | 0 | 0 | 0 |
| Downy brome | 10 | 0 | 0 | 0 | 0 | 0 |
| Cheat grass | 20 | 0 | 0 | 0 | 0 | 0 |
| Blackgrass | 90 | 90 | 80 | 80 | 60 | 20 |
| Ann. bluegrass | 20 | 20 | 0 | 0 | 0 | 0 |
| Green foxtail | 40 | 20 | 20 | 0 | 0 | 0 |
| Italian ryegrass | 60 | 50 | 40 | 20 | 0 | 0 |
| Rape | 100 | 100 | 100 | 100 | 100 | 100 |
| Winter wheat | 10 | 0 | 0 | 0 | 0 | 0 |
| Winter barley | 20 | 20 | 0 | 0 | 0 | 0 |
| Jointed goatgrass | 60 | 60 | 50 | 40 | 20 | 0 |
| Wild oat (Stg2) | 20 | 0 | 0 | 0 | 0 | 0 |
| Blackgrass (Stg2) | 80 | 80 | 80 | 80 | 70 | 40 |
| Ctchwd. bedstraw | 100 | 90 | 90 | 70 | 50 | 30 |
| Wild buckwheat | 50 | 50 | 30 | 10 | 0 | 0 |
| Kochia | 30 | 20 | 20 | 0 | 0 | 0 |
| Sntls. chamomile | 100 | 100 | 90 | 80 | 70 | 30 |
| Blk. nightshade | 60 | 60 | 40 | 40 | 20 | 20 |
| Russian thistle | - | - | - | - | - | - |
| Persn. speedweed | 40 | 30 | 10 | 10 | 0 | 0 |
| Sugarbeet | 100 | 100 | 90 | 80 | 70 | 60 |
| Ivylf. speedwell | 90 | 90 | 90 | 80 | 70 | 50 |
| Lambsquarters | 60 | 20 | 0 | 0 | 0 | 0 |
| Pennycress | 100 | 100 | 100 | 90 | 70 | 60 |
| Field violet | 80 | 50 | 40 | 20 | 0 | 0 |

TABLE C

CMPD 2

| Rate (g/ha) | 125 | 64 | 32 | 16 | 8 | 4 |
|---|---|---|---|---|---|---|
| PREEMERGENCE | | | | | | |
| Spring wheat | 20 | 10 | 10 | 0 | 0 | 0 |
| Spring barley | 60 | 50 | 40 | 20 | 10 | 0 |
| Wild oat | 0 | 0 | 0 | 0 | 0 | 0 |
| Downy brome | 40 | 20 | 0 | 0 | 0 | 0 |
| Cheat grass | 30 | 10 | 0 | 0 | 0 | 0 |
| Blackgrass | 100 | 90 | 80 | 60 | 20 | 0 |
| Ann. bluegrass | 80 | 70 | 40 | 30 | 0 | 0 |
| Green foxtail | 90 | 40 | 20 | 0 | 0 | 0 |
| Italian ryegrass | 70 | 70 | 40 | 30 | 10 | 0 |
| Rape | 100 | 100 | 100 | 100 | 80 | 70 |
| Winter wheat | 20 | 10 | 0 | 0 | 0 | 0 |
| Winter barley | 80 | 50 | 40 | 20 | 0 | 0 |
| Jointed goatgrass | 0 | 0 | 0 | 0 | 0 | 0 |
| Ctchwd. bedstraw | 90 | 90 | 80 | 50 | 30 | 20 |
| Wild buckwheat | 100 | 100 | 100 | 80 | 70 | 50 |
| Kochia | 50 | 50 | 40 | 40 | 20 | 0 |
| Sntls. chamomile | 100 | 90 | 90 | 80 | 70 | 60 |
| Blk. nightshade | 70 | 70 | 60 | 50 | 50 | 30 |
| Russian thistle | - | - | - | - | - | - |
| Persn. speedweed | 90 | 80 | 70 | 70 | 60 | 50 |
| Sugarbeet | 100 | 100 | 100 | 100 | 90 | 80 |
| Ivylf. speedwell | 90 | 80 | 80 | 70 | 50 | 20 |
| Lambsquarters | 90 | 90 | 90 | 80 | 70 | 60 |
| Pennycress | 100 | 100 | 100 | 90 | 90 | 80 |
| Field violet | 100 | 100 | 100 | 80 | 70 | 50 |

## Claims

1. A compound of the formula:

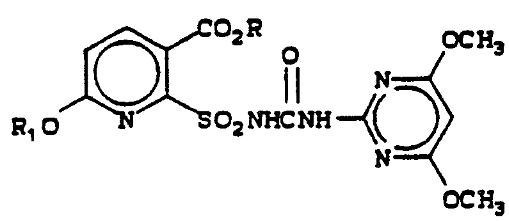 I

wherein
R is $C_1$-$C_3$ alkyl; and
$R_1$ is $C_1$-$C_2$ fluoroalkyl;
and their agriculturally suitable salts.

2. A compound of Claim 1 wherein when R is $CH_3$ or $CH_2CH_3$, then $R_1$ is other than $CH_2CF_3$, $CF_2H$ and $CF_3$.

3. The compound of Claim 1 which is methyl 2-[[[[(4,6-dimethoxypyrimidin-2-yl)amino]carbonyl]amino]-sulfonyl]-6-(2,2,2-trifluoroethoxy)-3-pyridinecarboxylate.

4. A composition suitable for controlling the growth of undesired vegetation which comprises an effective amount of a compound of the formula

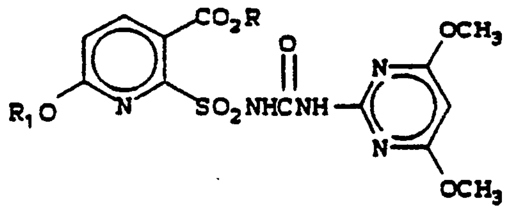

I

wherein
R is $C_1$-$C_3$ alkyl; and
$R_1$ is $C_1$-$C_2$ fluoroalkyl;
and their agriculturally suitable salts; and at least one of the following: surfactant, solid diluent or liquid diluent.

5. A composition as in Claim 5, wherein the effective compound is methyl 2-[[[[(4,6-dimethoxypyrimidin-2-yl)amino]carbonyl]amino]sulfonyl]-6-(2,2,2-trifluoroethoxy)-3-pyridinecarboxylate.

6. A method for controlling the growth of undesired vegetation which comprises applying to the locus to be protected an effective amount of a compound of the formula

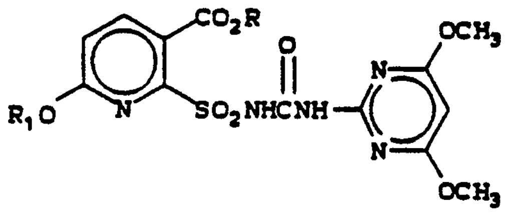

I

wherein
R is $C_1$-$C_3$ alkyl; and
$R_1$ is $C_1$-$C_2$ fluoroalkyl;
and their agriculturaLly suitable salts.

7. The method of Claim 6 wherein the substituent $R_1$ in the effective compound is $CF_2H$ or $CH_2CF_3$.

8. The method of Claim 6 wherein the effective compound is methyl 2-[[[[)4,6-dimethoxypyrimidin-2-yl)-amino]carbonyl]amino]sulfonyl]-6-(2,2,2-trifluoroethoxy)-3-pyridinecarboxylate.

European Patent Office

# EUROPEAN SEARCH REPORT

Application Number

EP 89 30 0726

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 162 723 (E.I. DU PONT DE NEMOURS AND CO.) * Pages 231,240,241 * | 1,4 | C 07 D 401/12<br>A 01 N 47/36 |
| A | EP-A-0 023 422 (E.I. DU PONT DE NEMOURS AND CO.) * Claims * | 1,4 | |
| | | | TECHNICAL FIELDS SEARCHED (Int. Cl.4) |
| | | | C 07 D 401/00 |

The present search report has been drawn up for all claims

| Place of search | Date of completion of the search | Examiner |
|---|---|---|
| THE HAGUE | 25-04-1989 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)